# EUROPÄISCHE PATENTSCHRIFT

(11) **EP 1 758 842 B1**
(45) Veröffentlichungstag und Bekanntmachung des Hinweises auf die Patenterteilung: **03.09.2014**
(21) Anmeldenummer: 05762836.4
(22) Anmeldetag: 22.06.2005
(51) Int. Cl.: C07C 29/54, C07C 31/125

(54) **VERFAHREN ZUR HERSTELLUNG PRIMÄRER LANGKETTIGER ALKOHOLE**
METHOD FOR THE PRODUCTION OF PRIMARY LONG-CHAIN ALCOHOLS
PROCEDE POUR PRODUIRE DES ALCOOLS PRIMAIRES A LONGUE CHAINE

(30) Priorität: 22.06.2004 DE 102004030080
(43) Veröffentlichungstag der Anmeldung: 07.03.2007
(73) Patentinhaber: SASOL Germany GmbH, 20537 Hamburg (DE)
(72) Erfinder: ZIEHE, Holger, 25524 Itzehoe (DE)
(74) Vertreter: Müller Schupfner & Partner Patent- und Rechtsanwaltspartnerschaft mbB
(86) Internationale Anmeldenummer: PCT/DE2005/001117
(87) Internationale Veröffentlichungsnummer: WO 2005/123639

(56) Entgegenhaltungen:
- EP-A- 0 577 020

## Beschreibung

Gegenstand der Erfindung ist ein Verfahren zur Herstellung primärer langkettiger Alkohole mit im Mittel größer 20 Kohlenstoffatomen.

Behenylalkohol ist ein linearer langkettiger Fettalkohol der Formel C₂₂H₄₅OH, jedoch werden auch Fettalkoholgemische mit einem Kettenlängenbereich von C₁₈ (Octadecanol) bis C₂₂ (Docosanol) als Behenylalkohole bezeichnet. Diese werden großtechnisch mittels Fettsäure- oder Fettsäureester-Hochdruckhydrierung hergestellt. Als Rohstoffe dienen hierbei insbesondere Eruca-, Crambe- oder Fischfettsäure, bzw. deren Ester, die durch Hydrolyse oder Umesterung der entsprechenden Fette gewonnen werden. Ein Nachteil dieses Verfahrens besteht darin, dass Rohstoffe aus natürlichen Quellen stets Fettsäuremischungen liefern, die Herstellung einer bestimmten Einzelkomponente somit grundsätzlich an die Anwesenheit weiterer Komponenten gekoppelt ist. Weiterhin ist dieses Verfahren aufgrund der natürlichen Verfügbarkeit der Fettsäuren bis auf wenige Ausnahmen auf Kettenlängen bis C₂₂ beschränkt.

Nach einem weiteren Verfahren sind Fettalkohole im Kettenlängenbereich von C₂₀ bis C₃₄ gemäß der US 2002/0099099-A1 erhältlich. Hierbei werden die Fettalkohol-Mischungen von natürlichen Produkten, vorzugsweise Bienenwachs, durch Extraktion und Aufreinigung in organischen Lösungsmitteln gewonnen. Alkohol-Ausbeuten von maximal 10 bis 15 % können erhalten werden, wenn die im Bienenwachs enthaltenen Ester vor der Extraktion verseift werden.

Ebenso werden langkettige Fettalkohole bis zu einer Kettenlänge von ca. C₃₀ ausgehend von Aluminium, Wasserstoff und Ethylen großtechnisch mittels Ziegler-Verfahren hergestellt (H. Ridder, K. Noweck, Ullmann's Encyclopedia of Industrial Chemistry, Fatty Alcohols, Fifth Edition, Vol. A10, 277-296 (1987)). Triethylaluminium wird hierbei mit Ethylen einer Wachstumsreaktion unterworfen, darunter wird die schrittweise Insertion von Ethylen in die Aluminiumalkylgruppe verstanden, und nach Oxidation zum Aluminium-Alkoxid und Hydrolyse werden C₂ bis C₃₀ Fettalkohol-Mischungen erhalten, wobei die Fettalkohole in einer Poisson-Verteilung vorliegen. Die Verteilungskurve weist bei den großtechnisch durchgeführten Verfahren ein Maximum bei C₁₀ bis C₁₂ auf, kann aber durch die Menge des eingesetzten Ethylens zu niedrigeren oder höheren mittleren Molekulargewichten verschoben werden. Die Rohalkohole werden anschließend destilliert und in Mischungen oder Einzelfraktionen bis zu einer Kettenlänge von C₁₈ bzw. C₂₀ aufgetrennt. Der anfallende Destillationssumpf enthält eine Alkoholverteilung mit einem Maximum bei C₂₀ und einem Alkoholanteil von ca. 80 Gew.-% bzw. einem Maximum bei C₂₂ bei einem Alkoholanteil von ca. 65 Gew.-%. Der bei dem Ziegler - Verfahren erhaltene Rohalkohol enthält Verunreinigungen, wie beispielsweise Paraffine, Olefine, Ether, Ester und Aldehyde.

Nach der US 3,255,256 werden Alkoholmischungen, wie beispielsweise im Ziegler-Prozeß nach Oxidation und Hydrolyse gewonnen, zu Aluminium-Alkoxiden umgesetzt, um dann die leichter flüchtigen Verunreinigungen durch Destillation oder Strippen abzutrennen. Auf diese Art gelingt die Abtrennung von Paraffinen, Olefinen, Ethern, Estern und Aldehyden von Alkoholen bis zu einer Kettenlänge, oberhalb derer aufgrund der hohen Siedepunkte der Nebenkomponenten eine thermische Zersetzung des Alkoxids stattfindet. Die anschließende Hydrolyse der Aluminiumalkoholate mit wässrigen Systemen führt zu einem Rohalkoholgemisch und Aluminiumhydroxid.

Dieses Verfahren ist anwendbar zur Abtrennung der nichtalkoholischen Nebenkomponenten des Ziegler-Verfahrens; es ist jedoch nicht geeignet, um lineare von verzweigten Alkoholen zu trennen. Der Ziegler - Rohalkohol enthält mit wachsender Kettenlänge auch einen steigenden Anteil an überwiegend 2-verzweigten Alkoholen. Die Analyse der Alkoholfraktion eines typischen Ziegleralkohol (NAFOL^{®} 20+) nach säulenchromatographischer Trennung ergibt beispielsweise bei einer Kettenlänge von C₂₀ 7,8 %, bei C₂₂ 8,0 % und bei C₂₄ bereits 17 % verzweigte Alkohole (Tabelle 1).

**Tabelle 1**

| *Beispiel einer Alkoholverteilung der Alkoholfraktion im NAFOL^{®} 20*+ | | | | |
|---|---|---|---|---|
| Kettenlänge | Bezeichnung des n-Alkohols | n-Alkohol Massen-[%] | iso-Alkohol Massen-[%] | Relativer Anteil iso-Alkohol [%] |
| C₁₈ | Octadecanol | 2 | 0< 0,1% | - |
| C₂₀ | Eicosanol | 40,4 | 3,4 | 7,8 |
| C₂₂ | Docosanol | 28,7 | 2,5 | 8,0 |
| C₂₄ | Tetracosanol | 8,8 | 1,8 | 17,0 |
| C₂₆ | Hexacosanol | 4,3 | 1,2 | 21,8 |
| C₂₈ | Octacosanol | 2,1 | 0,9 | 30,0 |
| C₃₀ | Triacontanol | 1,0 | 0,6 | 37,5 |
| C₃₂ | Dotriacontanol | 0,5 | 0,3 | 37,5 |
| Summe | | **87,8** | **10,7** | **10,9** |

Die EP 0577020 A1 offenbart ein Verfahren zur Herstellung von linearen Alkoholen mit einer Kettenlänge von 4 bis 22 Kohlenstoffatomen. Aluminiumalkyle mit Alkylresten von 2 bis 20 Kohlenstoffatomen und im Mittel zumindest 8 Kohlenstoffatomen werden als Startverbindungen in Anwesenheit eines Austausch-Katalysators mit α-Olefinen umgesetzt, wobei die α-Olefine um 2 Kohlenstoffatome länger sind und im Mittel zumindest 10 Kohlenstoffatome aufweisen. Durch die Umsetzung der Startverbindungen mit α-Olefinen werden die Alkylreste der Startverbindungen durch Austausch mit im Mittel um jeweils 2 Kohlenstoffatome längeren Olefine abgespalten, so dass Aluminium-Verbindungen mit Alkylresten von 4 bis 22 Kohlenstoffatomen entstehen, während die sich bildenden kurzkettigeren Oleline als Abspaltprodukte durch Unterdruck aus der Reaktionslösung entfernt werden. Die durch den Austausch erhaltenen Aluminium-Verbindungen werden zur Herstellung der Alkohole in weiteren Schritten zunächst oxidiert und anschließend hydrolysiert. Anders als die EP 0577020 A1 sieht die vorliegende Erfindung den Zusatz von Olefinen mittlerer Kettenlängen und eines AustauschKatalysators nicht vor und verwendet stattdessen Ethylen als Wachstumsolefin.

Bei der Wachstumsreaktion im Ziegler-Prozess wird Ethylen stufenweise in die Aluminium-Kohlenstoff-Bindung der Aluminiumalkyle insertiert. Ausgehend von Triethylaluminium werden längerkettige Aluminiumalkyle mit gerader Kohlenstoffanzahl gebildet. Eine der Nebenreaktionen ist die thermische Abspaltung von α-Olefinen, vorzugsweise ≥ C₄, unter gleichzeitiger Bildung eines Dialkylaluminiumhydrids. Die Olefinabspaltung ist eine Gleichgewichtsreaktion, d. h. dass das Dialkylaluminiumhydrid wiederum mit Ethylen oder den α-Olefinen mit Kettenlängen ≥ C₄ zum Trialkylaluminium reagieren kann. Ebenso wie bei der Wachstumsreaktion mit Ethylen können auch die gebildeten α-Olefine in die Aluminium-Kohlenstoff-Bindung insertieren. Diese Hydroaluminierungsreaktion erfolgt regioselektiv bevorzugt unter Ausbildung eines 2-alkylverzweigten Liganden, der wiederum bevorzugt unter Ausbildung eines verzweigten Olefins abgespalten wird. Verzweigte an das Aluminium gebundene Liganden können auch weiterhin mit Ethylen zu längeren Ketten wachsen, wobei sich die Verzweigungsstelle dann um jeweils zwei Kohlenstoffatome weiter vom Aluminiumatom entfernt.

Aufgabe der vorliegenden Erfindung ist es, ein Verfahren zur Herstellung linearer langkettiger Alkohole der Formel R-OH, wobei R für einen linearen Alkylrest CₙH₂ₙ₊₁ steht, mit n ≥ 20, vorzugsweise 20 bis 40, zur Verfügung zu stellen, dass die Nachteile des Standes der Technik überwindet und sowohl Einzelschnitte als auch Alkoholmischungen mit hoher Linearität, enger Verteilung und hoher Reinheit in guten Ausbeuten liefert.

Die Aufgabe wird erfindungsgemäß gelöst durch ein Verfahren zur Herstellung linearer langkettiger Fettalkohole oder Fettalkoholgemische gemäß Anspruch 1. Bevorzugte Ausführungsformen sind Gegenstand der Unteransprüche.

Das erfindungsgemäße Verfahren ist ein modifiziertes Ziegler-Verfahren, bei dem durch die Wahl bzw. Zugabe geeigneter Startkomponenten für die Wachstumsreaktion weniger Nebenprodukte als beim herkömmlichen Ziegler-Prozess gebildet werden. Die Nebenprodukte unterscheiden sich in ihren physikalischen Eigenschaften, wie beispielsweise Siedepunkt oder Schmelzpunkt, von denen der Zielprodukte und können daher leicht durch geeignete Trennstufen, beispielsweise durch Destillation oder Kristallisation, abgetrennt werden. Somit gelangt man zu langkettigen Fettalkoholen, die bislang nach dem Ziegler-Verfahren nicht in gleicher Reinheit erhalten werden konnten. Die Aluminium-Verbindungen sind i.d.R. Aluminiumalkyle.

Überraschenderweise wurde gefunden, dass durch das erfindungsgemäße Verfahren die Bildung von verzweigten Alkoholen im Kettenlängebereich größer gleich 20, vorzugsweise bis 40, deutlich reduziert werden kann, wenn die in den Patentansprüchen beschriebenen Startverbindungen eingesetzt werden.

Die Herstellung der Startverbindungen für die erfindungsgemäße Wachstumsreaktion kann entsprechend dem Stand der Technik aus Aluminium, Wasserstoff und einem 1-Olefin erfolgen. Während zur Herstellung von Triethylaluminium Ethylen als Olefinkomponente verwendet wird, werden zur Herstellung der höheren Aluminiumalkyle längerkettige Olefine oder auch Olefingemische verwendet. Alternativ lassen sich die höheren Aluminiumalkyle durch Umalkylierung mit kurzkettigen Aluminiumalkylen, vorzugsweise mit Tri(*iso*-butyl)aluminium oder Di(*iso*-butyl)aluminiumhydrid oder durch Addition von Olefinen an Di(n-alkyl)aluminiumhydrid, einer Zwischenstufe der Trialkylaluminium-Herstellung, herstellen. Die Nebenprodukte der Trialkylaluminium-Herstellung, insbesondere inerte Komponenten und/oder Aktivatoren aus dem metallischen Aluminium lassen sich durch Destillation oder Filtration; die Nebenprodukte der Umalkylierung, insbesondere dimere Olefine, mittels Kristallisation abtrennen.

Die erfindungsgemäße Wachstumsreaktion kann sowohl batchweise in einem Rührkesselreaktor aber auch kontinuierlich, beispielsweise in einem Strömungsrohr, in Gegenwart oder Abwesenheit von Lösungsmittel durch Aufpressen von Ethylen erfolgen. Vorzugsweise arbeitet man in einem Temperaturbereich von 100 bis 130 °C und einem Druck von 20 bis 120 bar. Nach Oxidation der Aluminiumalkyle mit Sauerstoff oder sauerstoffhaltigen Gasgemischen in Gegenwart oder Abwesenheit von Katalysatoren und anschließender Hydrolyse mit wässrigen Systemen, beispielsweise verdünnter Säure, erhält man nach Phasentrennung ein Fettalkoholrohprodukt, das - je nach Bedarf - in Alkoholeinzelschnitte oder Alkoholmischungen, vorzugsweise destillativ, aufgetrennt werden kann.

Nach einer zusätzlichen Option können die als Nebenprodukte der Wachstumsreaktion thermisch gebildeten langkettigen Olefine, überwiegend α-Olefine, vor der Oxidation durch Umsetzung mit kurzkettigen Aluminiumalkylen, beispielsweise Tri(isobutyl)aluminium, Di(*iso*-butyl)aluminiumhydrid oder langkettigen Di(n-alkyl)aluminiumhydrid mittels Ligandenaustausch, bzw. Additionsreaktion an das Aluminiumatom gebunden werden. Diese Umsetzung erfolgt thermisch, bevorzugt unter kontinuierlicher Ausschleusung der kurzkettigen Liganden und erhöht die Gesamtausbeute der langkettigen 1-Alkohole.

Nach einer weiteren zusätzlichen Option werden die langkettigen thermisch gebildeten 1-Olefine durch Umsetzung an kurzkettige Aluminiumalkyle gebunden, indem diese nicht zusätzlich zugefügt werden, sondern ein "in-situ" Austausch mit den bereits vorhandenen kurzkettigen Aluminiumalkylen unter kontinuierlicher Ausschleusung der kurzkettigen Olefine durchgeführt wird. Dieser Austausch wird bevorzugt in einer Destillations- oder Stripperkolonne durchgeführt. Der Ligandenaustausch führt im Endprodukt zu einer Verschiebung der Alkoholverteilung zu längerkettigeren Alkoholen.

Zur Verdeutlichung des erfindungsgemäßen Verfahrens wird im Folgenden die Wachstumsreaktion an Tri(octadecyl)aluminium mit Ethylen beispielhaft und auf andere entsprechende Edukte verallgemeinerbar beschrieben, die nach Oxidation und Hydrolyse zu einer Alkoholverteilung führt. Aus der direkten Hydrolyse des Wachstumsproduktes vor der Oxidation erhält man eine Paraffin- und Olefin-Verteilung. 1-Octadecen wird in Gegenwart oder Abwesenheit von Lösungsmitteln durch Ligandenaustauschreaktion mit Tri(iso-butyl)aluminium an das Aluminiumatom gebunden und man erhält Tri(octadecyl)aluminium, das zunächst durch Kristallisation aufgereinigt oder aber auch direkt in die Wachstumsreaktion mit Ethylen eingesetzt werden kann. Die Wachstumsreaktion erfolgt bei erhöhtem Ethylendruck von vorzugsweise 20 bis 120 bar und Temperaturen von vorzugsweise 100 bis 130 °C.

Nach Oxidation mit Sauerstoff und Hydrolyse mit wässrigen Systemen erhält man ein Rohalkohol-Gemisch mit einer Kettenlängenverteilung von überwiegend linearen C₁₈ bis C₃₂ Alkoholen, wobei als Nebenprodukte fast ausschließlich 1-Olefine und n-Paraffine und erstaunlicherweise jedoch keine *iso*-Alkohole in diesem Kettenlängenbereich detektiert werden können. Die gebildeten 1-Olefine sind Nebenprodukte der Wachstumsreaktion während die n-Paraffine größtenteils durch Hydrolyse der Aluminiumalkyle nach unvollständiger Oxidation gebildet werden. Bei diesem auf diese Art und Weise erhaltenen Produkt handelt es sich um ein Rohprodukt, dass nicht auf der Stufe des Alkoxides destilliert oder gestrippt wurde. Durch Strippen des Aluminium-Alkoxids wird das Verhältnis von n/iso-Alkohol nicht verändert, jedoch der Gehalt an Alkoholen deutlich erhöht.

**Tabelle 2**

| Kettenlänge | Bezeichung des n-Paraffins | n-Paraffin | iso-Paraffin | l-Olefin | iso-Olefin |
|---|---|---|---|---|---|
| | | [%] | [%] | [%] | [%] |
| C₆ | Hexan | 1,31 | n. d. | 0,11 | n. d. |
| C₈ | Octan | 6,85 | n. d. | 2,51 | n. d. |
| C₁₀ | Decan | 11,49 | 0,13 | 2,07 | 0,04 |
| C₁₂ | Dodecan | 10,27 | 0,10 | 1,17 | 0,04 |
| C₁₄ | Tetradecan | 6,01 | 0,01 | 0,48 | 0,00 |
| C₁₆ | Hexadecan | 2,59 | 0,09 | 0,18 | 0,17 |
| C₁₈ | Octadecan | 8,59 | 0,65* | 3,35 | 1,96* |
| C₂₀ | Eicosan | 11,49 | 0,05 | 2,13 | 0,03 |
| C₂₂ | Docosan | 8,97 | 0,02 | 1,03 | 0,03 |
| C₂₄ | Tetracosan | 4,71 | 0,02 | 0,39 | 0,01 |
| C₂₆ | Hexacosan | 1,86 | 0,01 | 0,13 | 0,01 |
| C₂₈ | Octacosan | 0,60 | 0,01 | 0,04 | 0,01 |
| C₃₀ | Triacontan | 0,17 | n. d. | 0,01 | n. d. |
| Summe | | **74,91** | **1,09** | **13,6** | **2,30** |

| | | | | | |
|---|---|---|---|---|---|
| * *iso*-Octadecan und *iso*-Octadecen im 1-Octadecen enthalten | | | | | |

Weitere Nebenprodukte sind kurzkettige Produkte, die aufgrund thermischer Olefinbildung in der Wachstumsreaktion gebildet werden. Die Abspaltung von 1-Olefinen führt zu Di(alkyl)aluminiumhydriden, die wiederum mit Ethylen und folgender Wachstumsreaktion zu kurzkettigen Aluminiumalkylen und folglich zu kurzkettigen Alkoholen reagieren können. Diese Nebenprodukte bilden sich in geringen Mengen, lassen sich aber aufgrund ihrer Siedelage aus dem Endprodukt leicht durch Destillation abtrennen.

Es können auch Gemische von Aluminiumalkylen eingesetzt werden. Ein Gemisch aus Tri(octyl)aluminium und Tri(octadecyl)aluminium führt nach Oxidation und Hydrolyse z.B. zu einer Alkoholverteilung mit zwei Maxima. Aus der direkten Hydrolyse des Wachstumsproduktes vor der Oxidation erhält man eine Paraffin- und Olefin-Verteilung gemäß Tabelle 2.

Nach der weiteren oben schon kurz erwähnten Option des erfindungsgemäßen Verfahrens können die während der Wachstumsreaktion nach dem erfindungsgemäßen

Verfahren thermisch gebildeten Olefine rückgewonnen werden. Dazu werden die langkettigen Olefine durch Ligandenaustauschreaktion mit kurzkettigen Aluminiumalkylen, beispielswesie Tri(*iso*-butyl)aluminium, wieder an das Aluminium gebunden und nach Oxidation und Hydrolyse erhält man ein Rohalkoholgemisch mit deutlich höheren n-Alkohol- und reduzierten 1-Olefin-Gehalt.

Ein zu langes Erwärmen in Gegenwart eines Überschusses an Tri(*iso*-butyl)aluminium führt zu einer erhöhten Aluminiumhydrid-Bildung und somit durch "in-situ" -Hydrolyse zu einer erhöhten Paraffinbildung in der Oxidationsstufe. Ein erhöhter Hydridgehalt kann allerdings auch vor der Oxidationsstufe durch Zugabe von 1-Olefinen erniedrigt werden.

### Beispiele

### 1.A Herstellung von Tri(octadecyl)aluminium

29,3 g (116 mol, 90 %ig) 1-Octadecen und 26,87 g (d = 0,695 g/mol; 38,7 ml; 38,7 mmol) einer 1-molaren Tri(*iso*-butyl)aluminium-Lösung in Hexan wurden unter Stickstoffabdeckung in einem 150-ml-Schlenckgefäß gemischt und das Hexan abdestilliert. Das Gemisch wurde weitere 8,25 Stunden auf 125 bis 135 °C erwärmt, wobei das gebildete gasförmige Isobuten über einen Blasenzähler ausgeschleust wurde. Nach dem Abkühlen auf 20 °C erhielt man das Tri(octadecyl)aluminium als weißen Feststoff. Mittels NMR-Analyse wurden, bezogen auf 100 % Aluminiumalkyl (Al-CH₂-), 1,5 % Dialkylaluminiumhydrid (Al-H), 2 % *alpha-* (RCH=CH₂) und 3,8 % interne Olefine (RHC=CHR') gemessen. Das Tri(octadecyl)aluminium wurde mit 50 g Toluol verdünnt und als Startverbindung direkt für die folgende Wachstumsreaktionen verwendet (1.C).

### 1.B Kristallisation von Tri(octadecyl)aluminium (wahlweise)

80,0 g Tri(octadecyl)aluminium und 300 ml Pentan wurden unter Rückfluss erhitzt. Es wurde sukzessive soviel Pentan zugegeben, bis eine klare, gesättigte Lösung entstanden war. Anschließend wurde die Lösung auf 20 °C abgekühlt. Die über dem Feststoff stehende flüssige Phase wurde abdekantiert. Nach erneuter Kristallisation nach dem gleichen Verfahren erhielt man einen weißen Feststoff. Die Analyse des hydrolysierten Produktes zeigte einen Anstieg des n-Octadecan-Gehaltes von 77,6 auf 95,8 %. Weiterhin wurden 1,0 % n-Hexadecan und 0,5 % n-Eicosan detektiert.

### 1.C Wachstumsreaktion an Tri(octadecyl)aluminium mit Ethylen

Jeweils 20 ml der in Versuch 1.A hergestellten Tri(octadecyl)aluminium-Lösung in Toluol wurden in 150-ml-Stahlbomben bei 20 °C mit 23,8 bar, bzw. 35 bar Ethylendruck versehen und die Bomben anschließend in einem Rollenofen auf 116 °C erwärmt. Im warmen Zustand stellte sich zunächst ein Druck von etwa 35 bar (bzw. 56 bar) ein, der während der Reaktion aufgrund der Ethylenaufnahme leicht sank. Nach Ablauf der Reaktionszeit von 2 bis 3 Stunden und anschließendem Abkühlen auf ca. 40 °C wurde die Stahlbombe in der "Glovebox" entspannt und jeweils eine Probe mit 10%iger Schwefelsäure hvdrolvsiert und analysiert.

**Tabelle 3**

| **Zusammensetzung^{#} des Wachstumsproduktes von Tri(octadecyl)aluminium nach anschließender Hydrolyse.** | | | | | |
|---|---|---|---|---|---|
| **a) 23,8 bar (kalt), 120 min** | | | | | |
| Kettenlänge | Bezeichnung des n-Paraffins | n-Paraffin | iso-Paraffin | l-Olefin | iso-Olefin |
| | | [%] | [%] | [%] | [%] |
| C₁₈ | Octadecan | 26,39 | 1,71* | 11,83 | 3,74* |
| C₂₀ | Eicosan | 22,25 | 0,08 | 5,00 | 0,04 |
| C₂₂ | Docosan | 9,99 | 0,02 | 1,46 | 0,04 |
| C₂₄ | Tetracosan | 3,02 | 0,01 | 0,33 | 0,01 |
| C₂₆ | Hexacosan | 0,69 | n. d. | 0,06 | n. d. |
| C₂₈ | Octacosan | 0,13 | n. d. | 0,01 | n. d. |
| C₃₀ | Triacontan | 0,02 | n. d. | n. d. | n. d. |
| Summe | | **62,49** | **1,82** | **18,69** | **3,83** |

| b) 23,8 bar (kalt), 180 min | | | | | |
|---|---|---|---|---|---|
| Kettenlänge | Bezeichnung des n-Paraffins | n-Paraffin | iso-Paraffin | l-Olefin | iso-Olefin |
| | | [%] | [%] | [%] | [%] |
| C₁₈ | Octadecan | 10,19 | 0,92* | 15,83 | 4,13* |
| C₂₀ | Eicosan | 13,39 | 0,05 | 10,47 | 0,05 |
| C₂₂ | Docosan | 10,60 | 0,03 | 5,01 | 0,05 |
| C₂₄ | Tetracosan | 5,71 | 0,03 | 1,89 | 0,02 |
| C₂₆ | Hexacosan | 2,33 | 0,01 | 0,58 | 0,01 |
| C₂₈ | Octacosan | 0,77 | 0,01 | 0,16 | 0,01 |
| C₃₀ | Triacontan | 0,22 | n. d. | 0,04 | n. d. |
| Summe | | **43,21** | **1,05** | **33,98** | **4,27** |
| | | | | | |

| c) 35 bar (kalt), 125 min | | | | | |
|---|---|---|---|---|---|
| Kettenlänge | Bezeichnung des n-Paraffins | n-Paraffin | iso-Paraffin | l-Olefin | iso-Olefin |
| | | [%] | [%] | [%] | [%] |
| C₁₈ | Octadecan | 19,31 | 1,25* | 7,52 | 3,95* |
| C₂₀ | Eicosan | 22,95 | 0,06 | 3,07 | 0,07 |
| C₂₂ | Docosan | 15,63 | 0,02 | 1,33 | 0,03 |
| C₂₄ | Tetracosan | 7,12 | n. d. | 0,46 | n. d. |
| C₂₆ | Hexacosan | 2,48 | n. d. | 0,15 | n. d. |
| C₂₈ | Octacosan | 0,72 | n. d. | 0,04 | n. d. |
| C₃₀ | Triacontan | 0,19 | n. d. | 0,01 | n. d. |
| Summe | | **68,40** | **1,33** | **12,58** | **4,05** |
| | | | | | |

| d) 35 bar (kalt), 145 min | | | | | |
|---|---|---|---|---|---|
| Kettenlänge | Bezeichnung des n-Paraffins | n-Paraffin | iso-Paraffin | l-Olefin | iso-Olefin |
| | | [%] | [%] | [%] | [%] |
| C₁₈ | Octadecan | 13,26 | 1,06* | 8,72 | 4,07* |
| C₂₀ | Eicosan | 18,25 | 0,04 | 4,40 | 0,06 |
| C₂₂ | Docosan | 15,49 | 0,02 | 2,29 | 0,03 |
| C₂₄ | Tetracosan | 9,00 | 0,02 | 1,02 | 0,01 |
| C₂₆ | Hexacosan | 4,20 | n. d. | 0,42 | n. d. |
| C₂₈ | Octacosan | 1,71 | n. d. | 0,18 | n. d. |
| C₃₀ | Triacontan | 0,69 | n. d. | 0,08 | n. d. |
| Summe | | **62,60** | **1,14** | **17,11** | **4,17** |

| | | | | | |
|---|---|---|---|---|---|
| ^{#} ohne Lösungsmittel * Verunreinigungen aus dem 1-Octadecen enthalten n. d.: nicht detektiert | | | | | |

### 1.D Umsetzung des Wachstumsproduktes mit Tri(iso-butyl)aluminium

Die vereinigten Produkte aus Versuch 1.C.c und 1.C.d wurden mit 480 mg (0,69 ml) einer 1M-Lösung Tri(*iso*-butyl)aluminium in Hexan versetzt, nach Abdestillieren des Hexans weitere 5 Stunden auf 130 °C und 3 Stunden auf 110 °C erwärmt. Mittels NMR-Analyse war der α-Olefin-Gehalt (relativ zu Al-CH₂- =100) von 14,1 auf 7,8 % gesunken. Es wurden weitere 510 mg (0,73 ml) der Tri(*iso*-butyl)aluminium-Lösung zugegeben und weitere 4 Stunden auf 130 °C und 1 Stunde auf 110 °C erwärmt. Der α-Olefin-Gehalt lag nun bei 4,6 % (Tabelle 4).

**Tabelle 4**

| Zusammensetzung^{#} des Wachstumsproduktes nach Umsetzung mit Tri(*iso*-butyl)aluminium und anschließender Hydrolyse. | | | | | |
|---|---|---|---|---|---|
| Kettenlänge | Bezeichnung des n-Paraffins | n-Paraffin | iso-Paraffin | l-Olefin | iso-Olefin |
| | | [%] | [%] | [%] | [%] |
| C₁₈ | Octadecan | 22,54 | 2,25* | 1,35 | 2,81* |
| C₂₀ | Eicosan | 23,22 | 0,06 | 1,37 | 0,06 |
| C₂₂ | Docosan | 16,95 | 0,01 | 0,98 | 0,04 |
| C₂₄ | Tetracosan | 8,85 | 0,02 | 0,52 | 0,02 |
| C₂₆ | Hexacosan | 3,71 | n. d. | 0,24 | 0,02 |
| C₂₈ | Octacosan | 1,38 | n. d. | 0,10 | n. d. |
| C₃₀ | Triacontan | 0,50 | n. d. | 0,04 | n. d. |
| Summe | | **77,15** | **2,34** | **4,6** | **2,95** |

### 1.E Wachstumsreaktion an Gemischen aus Tri(octyl)aluminium und Tri(octadecyl)aluminium

10 ml der in Versuch 1.A hergestellten Tri(octadecyl)aluminium-Lösung in Toluol und 2,5 g Tri(octyl)aluminium in 2.32 g Toluol wurden in eine 150-ml-Stahlbombe gefüllt und der Druck bei 20 °C mit Ethylen auf 35 bar erhöht. Die Bombe wurde anschließend in einem Rollenofen 140 min auf 116 °C erwärmt. Im warmen Zustand stellte sich zunächst ein Druck von etwa 56 bar ein, der während der Reaktion aufgrund der Ethylenaufnahme sank. Nach Ablauf der Reaktionszeit und anschließendem Abkühlen auf ca. 40 °C wurde die Stahlbombe in der Glovebox entspannt, eine Probe mit 10%iger Schwefelsäure hydrolysiert und analysiert (Tabelle 5).

**Tabelle 5**

| Zusammensetzung^{#} des Wachstuinsproduktes von Gemischen aus Tri(octyl)aluminium und Tri(octadecyl)aluminium nach anschließender Hydrolyse. | | | | | |
|---|---|---|---|---|---|
| Kettenlänge | Bezeichung des n-Paraffins | n-Paraffin | iso-Paraffin | l-Olefin | iso-Olefin |
| | | [%] | [%] | [%] | [%] |
| C₆ | Hexan | 1,31 | n. d. | 0,11 | n. d. |
| C₈ | Octan | 6,85 | n. d. | 2,51 | n. d. |
| C₁₀ | Decan | 11,49 | 0,13 | 2,07 | 0,04 |
| C₁₂ | Dodecan | 10,27 | 0,10 | 1,17 | 0,04 |
| C₁₄ | Tetradecan | 6,01 | 0,01 | 0,48 | 0,00 |
| C₁₆ | Hexadecan | 2,59 | 0,09 | 0,18 | 0,17 |
| C₁₈ | Octadecan | 8,59 | 0,65* | 3,35 | 1,96* |
| C₂₀ | Eicosan | 11,49 | 0,05 | 2,13 | 0,03 |
| C₂₂ | Docosan | 8,97 | 0,02 | 1,03 | 0,03 |
| C₂₄ | Tetracosan | 4,71 | 0,02 | 0,39 | 0,01 |
| C₂₆ | Hexacosan | 1,86 | 0,01 | 0,13 | 0,01 |
| C₂₈ | Octacosan | 0,60 | 0,01 | 0,04 | 0,01 |
| C₃₀ | Triacontan | 0,17 | n. d. | 0,01 | n. d. |
| Summe | | **74,91** | **1,09** | **13,6** | **2,30** |

### 2.A Oxidation der vereinigten Fraktionen aus Versuch 1.C.a und 1.C.b

Die vereinigten Rohprodukte aus Versuch 1.C.a und 1.C.b wurden in einem 300ml Parr-Autoklav mit einem Stickstoffvordruck von 2,1 bar versehen. Bei 30 °C und einer Rührgeschwindigkeit von 650 U/min wurde Sauerstoff mit einer Flussrate von 23 ml/min zudosiert. Der Druck stieg innerhalb von 60 min auf 4,9 bar an, die Sauerstoffzufuhr wurde gestoppt, die Lösung weitere 2 Stunden auf 50 °C erwärmt und anschließend mit 10%iger Schwefelsäure hydrolysiert. Die in Tabelle 6 dargestellte Produktzusammensetzung wurde erhalten.

### 2.B Oxidation der vereinigten Fraktionen aus Versuch 1.D

Das Rohprodukt aus Versuch 1.D wurde in einem 300ml Parr-Autoklav mit einem Stickstoffvordruck von 2,1 bar versehen. Bei 30 °C und einer Rührgeschwindigkeit von 650 U/min wurde Sauerstoff mit einer Flussrate von 23 ml/min zudosiert. Nach 30 min Reaktionszeit wurden 6,6 mg Tetra(*iso*-propyl)titanat in 0,8 ml Tridecan zugegeben.

**Tabelle 6**

| Zusammensetzung^{#} des Wachstumsproduktes nach Oxidation und anschließender Hydrolyse | | | | | |
|---|---|---|---|---|---|
| Kettenlänge | Bezeichnung des n-Alkohols | n-Alkohol Massen-[%] | iso-Alkohol Massen-[%] | l-Olefin [%] | n-Paraffin [%] |
| C₁₈ | Octadecanol | 12,4 | n. d. | 15,4 | 3.5 |
| C₂₀ | Eicosanol | 13,4 | n. d. | 9,1 | 2,9 |
| C₂₂ | Docosanol | 7,8 | n. d. | 3,7 | 1,3 |
| C₂₄ | Tetracosanol | 3,2 | n. d. | 1,4 | 0,6 |
| C₂₆ | Hexacosanol | 1,0 | n. d. | 0,4 | 0,2 |
| C₂₈ | Octacosanol | 0,2 | n. d. | 0,1 | 0,08 |
| C₃₀ | Triacontanol | 0,05 | n. d | 0,05 | 0,03 |
| Summe | | **38,1** | **-** | **30,2** | **8,6** |

Der Druck stieg innerhalb von 52 min auf 5 bar an, die Sauerstoffzufuhr wurde gestoppt, die Lösung weitere 6 Stunden auf 50 °C erwärmt und anschließend mit 10%iger Schwefelsäure bei 80 °C hydrolysiert. Es wurde die in Tabelle 7 dargestellte Produktverteilung erhalten:

**Tabelle 7**

| Zusammensetzung^{#} des Wachstumsproduktes nach Oxidation und anschließender Hydrolyse. | | | | | |
|---|---|---|---|---|---|
| Kettenlänge | Bezeichung des n-Alkohols | n-Alkohol Massen-[%] | iso-Alkohol Massen-[%] | l-Olefin* [%] | n-Paraffin* [%] |
| C₁₈ | Octadecanol | 17,66 | n. d. | 1,87 | 5,83 |
| C₂₀ | Eicosanol | 19,46 | n. d. | 1,92 | 3,76 |
| C₂₂ | Docosanol | 13,62 | n. d. | 1,41 | 2,40 |
| C₂₄ | Tetracosanol | 6,93 | n. d. | 0,81 | 1,25 |
| C₂₆ | Hexacosanol | 2,04 | n. d. | 0,40 | 0,56 |
| C₂₈ | Octacosanol | 0,48 | n. d. | 0,22 | 0,24 |
| C₃₀ | Triacontanol | 0,10 | n. d | 0,13 | 0,12 |
| Summe | | **60,29** | **-** | **6,67** | **14,16** |

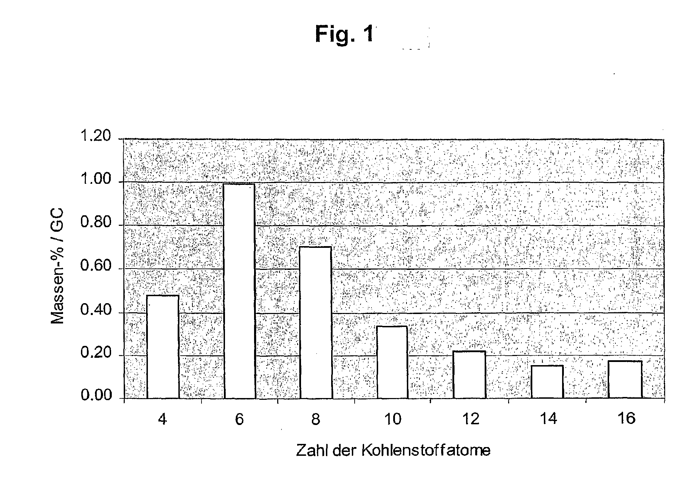

Eine Übersicht über die Nebenproduktbildung ist in Fig. 1 dargestellt (Alkoholverteilung).

Aus den gaschromatographischen Analysen wurde der Lösungsmittelanteil herausgerechnet. Die Bezeichnung n. d. (nicht detektiert) gibt an, dass die Produkte nicht vorhanden sind oder unterhalb der Nachweisgrenze liegen.

Die verwendeten Chemikalien wurden von der Firma Aldrich bezogen und direkt ohne weitere Reinigung eingesetzt. Das Tri(iso-butyl)aluminium ist eine 1-molare Lösung in Hexan. Das verwendete 1-Octadecen ist technischer Qualität und hat folgende Zusammensetzung: 1-Octadecen: 90,6 Gew.%; Vinyliden-Olefine: 4,2 Gew.%; interne Olefine:2,3 Gew.%; 1-Eicosene: 0,6 Gew.%; 1-Hexadecene: 0,4 Gew.%; andere Verbindungen: 1,9 Gew.%)

## Patentansprüche

1. Verfahren zur Herstellung primärer langkettiger Alkohole oder deren Mischungen mit im Mittel n Kohlenstoffatomen, wobei n größer gleich 20 ist, in einer Wachstumsreaktion, wobei das Verfahren folgende Schritte umfasst:
(a) Bereitstellen von Aluminium-Verbindungen als Startverbindungen der Wachstumsreaktion, die jeweils zumindest einen Kohlenwasserstoffrest mit zumindest (n/2 + 2) Kohlenstoffatomen aufweisen, wobei der Kohlenwasserstoffrest jeweils über ein primäres Kohlenstoffatom an das Aluminiumatom gebunden ist und wobei die Zusammensetzung der Startverbindungen vor der Wachstumsreaktion einen Gehalt von kleiner 10 Gew.-% an 1-Olefinen mit größer 2 und kleiner (n/2 + 2) Kohlenstoffatomen in der Summe aufweist,
(b) nachfolgendes Zusammenbringen der Startverbindungen in einer Wachstumszusammensetzung, enthaltend ggf. andere Aluminium-Verbindungen, mit Ethylen zur Umsetzung mit Ethylen unter Bildung von zumindest Aluminium-Verbindungen als Wachstumsverbindungen, die jeweils gegenüber der Startverbindung zumindest einen Kohlenwasserstoffrest mit im Mittel zumindest z + (n/2 + 2) Kohlenstoffatomen aufweisen, wobei z größer gleich 2 ist, wobei die Kohlenwasserstoffreste der Start- und Wachstumsverbindungen mit zumindest (n/2 + 2) Kohlenstoffatomen in der Summe zu zumindest 90 mol% linear sind,
(c) Oxidation der Umsetzungsprodukte mit Ethylen mit Sauerstoff, um Aluminiumoxy-Verbindungen mit zumindest einem über Sauerstoff an das Aluminium-Atom gebundenen Kohlenwasserstoffrest zu erhalten,
(d) Hydrolyse der Aluminiumoxy-Verbindungen, und
(e) Abtrennen einer Zusammensetzung enthaltend im Mittel Alkohole mit größer gleich 20 Kohlenstoffatomen.

2. Verfahren gemäß einem der vorhergehenden Ansprüche, **dadurch gekennzeichnet, dass** die Zusammensetzung der Startverbindungen vor der Wachstumsreaktion einen Gehalt von kleiner 3 Gew.-% an 1-Olefinen mit größer 2 und kleiner (n/2 + 2) Kohlenstoffatomen in der Summe aufweist.

3. Verfahren gemäß einem der vorhergehenden Ansprüche, **dadurch gekennzeichnet, dass** die Kohlenwasserstoffreste der Start- und Wachstumsverbindungen mit zumindest (n/2 + 2) Kohlenstoffatomen Alkylreste sind.

4. Verfahren gemäß einem der vorhergehenden Ansprüche, **dadurch gekennzeichnet, dass** die Zusammensetzung nach Hydrolyse der Aluminiumoxy-Verbindungen (Schritt (d)), bezogen auf alle Alkohole in der Zusammensetzung, zumindest 4 Gew.% Alkohole mit mehr als 20 KohlenstoffAtomen aufweist.

5. Verfahren gemäß einem der Ansprüche 1 bis 3, **dadurch gekennzeichnet, dass** die Zusammensetzung nach Hydrolyse der Aluminiumoxy-Verbindungen (Schritt (d)), bezogen auf alle Alkohole in der Zusammensetzung, zumindest 6 Gew.% Alkohole mit mehr als 20 Kohlenstoff-Atomen aufweist.

6. Verfahren gemäß einem der vorhergehenden Ansprüche, **dadurch gekennzeichnet, dass** die Alkohole mit mehr als 20 Kohlenstoffatomen durch Abtrennen der wässrigen Phase von der organischen Phase und/oder Destillation abgetrennt werden.

7. Verfahren gemäß zumindest einem der vorhergehenden Ansprüche, **dadurch gekennzeichnet, dass** der Gehalt an 1-Olefinen und/oder an Aluminium gebundenen Kohlenwasserstoffresten in der Wachstumszusammensetzung, die jeweils weniger als 24 Kohlenstoffatome aufweisen und verzweigt sind, in der Summe kleiner 5 Gew.% beträgt.

8. Verfahren gemäß einem der vorhergehenden Ansprüche, **dadurch gekennzeichnet, dass** die Startverbindungen durch Ligandenaustausch mit Aluminiumalkylen aufweisend Kohlenwasserstoffreste mit 6 oder weniger Kohlenstoffatomen in Gegenwart zumindest eines linearen langkettigen α-Olefins mit zumindest (n/2 + 2) Kohlenstoffatomen hergestellt sind.

9. Verfahren gemäß einem der Ansprüche 1 bis 7, **dadurch gekennzeichnet**, die Startverbindungen durch Umsetzung von Aluminium, Wasserstoff und zumindest einem linearen langkettigen α-Olefin mit zumindest (n/2 + 2) Kohlenstoffatomen hergestellt sind.

10. Verfahren gemäß einem der Ansprüche 1 bis 7, **dadurch gekennzeichnet**, die Startverbindungen durch Addition von α-Olefinen mit zumindest (n/2 + 2) Kohlenstoffatomen an AluminiumVerbindung ausweisend zumindest einen Kohlenwasserstoffrest und zumindest ein hydridisch gebundenes Wasseratom hergestellt sind.

11. Verfahren gemäß einem der vorhergehenden Ansprüche, **dadurch gekennzeichnet**, die Startverbindungen kristallisiert werden bevor sie der Wachstumszusammensetzung beigefügt werden.

12. Verfahren gemäß einem der vorhergehenden Ansprüche, **dadurch gekennzeichnet, dass** vor der Oxidation der Umsetzungsprodukte mit Sauerstoff, die in der Wachstumszusammensetzung enthaltenen α-Olefine mit zumindest (n/2 + 2) Kohlenstoffatomen an Aluminium-Verbindungen unter Abspaltung von α-Olefinen mit kleiner (n/2 + 2) Kohlenstoffatomen, vorzugsweise kleiner gleich 6 Kohlenstoffatomen, gebunden werden, vorzugsweise durch Temperaturerhöhung und Entfernen der Olefine mit kleiner (n/2 + 2) Kohlenstoffatomen aus dem Reaktionsgemisch.

13. Verfahren gemäß Anspruch 12, **dadurch gekennzeichnet, dass** die Aluminium-Verbindungen, die unter Abspaltung von α-Olefinen mit kleiner (n/2 + 2) reagieren, Di(iso C3- bis C6- alkyl)aluminiumhydrid oder Tri(iso C3- bis C6- alkyl)aluminium, vorzugsweise Di(isobutyl)aluminiumhydrid oder Tri(iso-butyl)aluminium, sind und diese ggf. zugesetzt werden.

14. Verfahren gemäß einem der vorhergehenden Ansprüche, **dadurch gekennzeichnet, dass** die Aluminiumoxy-Verbindungen vor der Hydrolyse destilliert oder gestrippt werden, um Nebenprodukte wie Paraffine, Olefine und/oder Ester aus dem Produktgemisch zu entfernen, während die Aluminiumoxy-Verbindungen im Sumpf verbleiben.

15. Verfahren gemäß einem der vorhergehenden Ansprüche, **dadurch gekennzeichnet, dass** die Wachstumsreaktion bei 100 bis 130 °C und einem Druck von 20 bis 120 bar durchgeführt wird.

16. Verfahren gemäß einem der vorhergehenden Ansprüche, **dadurch gekennzeichnet, dass** das Abtrennen gemäß Schritt (e) durch Destillation erfolgt und durch Destillation eine Alkoholfraktion gewonnen wird, die zu mehr als 90 mol%, insbesondere mehr als 95 mol%, lineare Alkohole enthält.

17. Verfahren gemäß einem der Ansprüche 1 bis 15, **dadurch gekennzeichnet, dass** als Startverbindungen folgende Aluminium-Verbindungen eingesetzt werden,
(I) Startverbindungen mit jeweils zumindest einem Kohlenwasserstoffrest mit größer gleich 12 Kohlenstoffatomen, vorzugsweise zumindest 2 und insbesondere 3 Resten, zur Herstellung einer Alkoholfraktion mit im Mittel 20 Kohlenstoffatomen;
(II) Startverbindungen mit jeweils zumindest einem Kohlenwasserstoffrest mit größer gleich 14 Kohlenstoffatomen, vorzugsweise zumindest 2 und insbesondere 3 Resten, zur Herstellung einer Alkoholfraktion mit im Mittel 20 bis 24 Kohlenstoffatomen;
(III) Startverbindungen mit jeweils zumindest einem Kohlenwasserstoffrest mit größer gleich 16 Kohlenstoffatomen, vorzugsweise zumindest 2 und insbesondere 3 Resten, zur Herstellung einer Alkoholfraktion mit im Mittel 20 bis 28 Kohlenstoffatomen;
(IV) Startverbindungen mit jeweils zumindest einem Kohlenwasserstoffrest mit größer gleich 18 Kohlenstoffatomen, vorzugsweise zumindest 2 und insbesondere 3 Resten, zur Herstellung einer Alkoholfraktion mit im Mittel 20 bis 32 Kohlenstoffatomen; oder
(V) Startverbindungen mit jeweils zumindest einem Kohlenwasserstoffrest mit größer gleich 20 Kohlenstoffatomen, vorzugsweise zumindest 2 und insbesondere 3 Resten, zur Herstellung einer Alkoholfraktion mit im Mittel 20 bis 36 Kohlenstoffatomen;
wobei das Abtrennen gemäß Schritt (e) durch Destillation erfolgt und
durch Destillation eine Alkoholfraktion gewonnen wird, die zu mehr als 90 mol%, insbesondere mehr als 95 mol%, lineare Alkohole enthält.

18. Verfahren gemäß einem der Ansprüche 1 bis 15, **dadurch gekennzeichnet, dass** als Startverbindungen folgende Aluminium-Verbindungen eingesetzt werden,
(II) Startverbindungen mit jeweils zumindest einem Kohlenwasserstoffrest mit größer gleich 14 Kohlenstoffatomen, vorzugsweise zumindest 2 und insbesondere 3 Resten, zur Herstellung einer Alkoholfraktion mit im Mittel 22 bis 24 Kohlenstoffatomen;
(III) Startverbindungen mit jeweils zumindest einem Kohlenwasserstoffrest mit größer gleich 16 Kohlenstoffatomen, vorzugsweise zumindest 2 und insbesondere 3 Resten, zur Herstellung einer Alkoholfraktion mit im Mittel 26 bis 28 Kohlenstoffatomen;
(IV) Startverbindungen mit jeweils zumindest einem Kohlenwasserstoffrest mit größer gleich 18 Kohlenstoffatomen, vorzugsweise zumindest 2 und insbesondere 3 Resten, zur Herstellung einer Alkoholfraktion mit im Mittel 30 bis 32 Kohlenstoffatomen; oder
(V) Startverbindungen mit jeweils zumindest einem Kohlenwasserstoffrest mit größer gleich 20 Kohlenstoffatomen, vorzugsweise zumindest 2 und insbesondere 3 Resten, zur Herstellung einer Alkoholfraktion mit im Mittel 34 bis 36 Kohlenstoffatomen;
wobei das Abtrennen gemäß Schritt (e) durch Destillation erfolgt und
durch Destillation eine Alkoholfraktion gewonnen wird, die zu mehr als 90 mol%, insbesondere mehr als 95 mol%, lineare Alkohole enthält.

## Claims

1. A method for preparing primary long-chain alcohols or mixtures thereof having on average n carbon atoms, wherein n is greater than or equal to 20, in a growth reaction, the method comprising the following steps:
(a) providing aluminum compounds as starting compounds of the growth reaction, each having at least one hydrocarbon group with at least (n/2 + 2) carbon atoms, each hydrocarbon group being bound to the aluminum atom via a primary carbon atom and wherein the composition of the starting compounds has in total a content of less than 10 wt% of 1-olefins having greater than 2 and less than (n/2 + 2) carbon atoms prior to the growth reaction;
(b) subsequently bringing together the starting compounds in a growth composition, that optionally contains other aluminum compounds, with ethylene for the conversion with ethylene to form at least aluminum compounds as growth compounds, which each have, compared to the starting compound, at least one hydrocarbon group with on average at least z + (n/2 + 2) carbon atoms, wherein z is greater than or equal to 2, wherein of the hydrocarbon groups of the starting and growth compounds having at least (n/2 + 2) carbon atoms at least 90 mol% in total are linear;
(c) oxidizing the conversion products with ethylene with oxygen in order to obtain aluminum oxy compounds having at least one hydrocarbon group that is bound to the aluminum atom via oxygen;
(d) hydrolysing of the aluminum oxy compounds; and
(e) separating a composition containing on average alcohols having greater than or equal to 20 carbon atoms.

2. The method according to any of the previous claims, **characterized in that** the composition of the starting compounds prior to the growth reaction has in total a content of less than 3 wt% of 1-olefins having greater than 2 and less than (n/2 + 2) carbon atoms.

3. The method according to any of the previous claims, **characterized in that** the hydrocarbon groups of the starting and growth compounds having at least (n/2 + 2) carbon atoms are alkyl groups.

4. The method according to any of the previous claims, **characterized in that** the composition after hydrolysis of the aluminum oxy compounds (step (d)), based on all alcohols in the composition, has at least 4 wt%, of alcohols with more than 20 carbon atoms.

5. The method according to one of the previous claims 1 to 3, **characterized in that** the composition after hydrolysis of the aluminum oxy compounds (step (d)), based on all alcohols in the composition, has at least 6 wt%, of alcohols with more than 20 carbon atoms.

6. The method according to any of the previous claims, **characterized in that** the alcohols with more than 20 carbon atoms are separated by separating the aqueous phase from the organic phase and/or by distillation.

7. The method according to at least one of the previous claims, **characterized in that** the content of 1-olefins and/or hydrocarbon groups bound to aluminum in the growth composition that contain fewer than 24 carbon atoms each and are branched is in total less than 5 wt%.

8. The method according to any of the previous claims, **characterized in that** the starting compounds are prepared by ligand exchange with aluminum alkyls having hydrocarbon groups with 6 or fewer carbon atoms in the presence of at least one linear long-chain α-olefin having at least (n/2 + 2) carbon atoms.

9. The method according to any of claims 1 to 7, **characterized in that** the starting compounds are prepared by conversion of aluminum, hydrogen, and at least one linear long-chain α-olefin having at least (n/2 + 2) carbon atoms.

10. The method according to any of claims 1 to 7, **characterized in that** the starting compounds are prepared by addition of α-olefins having at least (n/2 + 2) carbon atoms to an aluminum compound having at least one hydrocarbon group and at least one hydridically bound hydrogen atom.

11. The method according to any of the previous claims, **characterized in that** the starting compounds are crystallized prior to being incorporated into the growth composition.

12. The method according to any of the previous claims, **characterized in that** prior to oxidation of the conversion products with oxygen, the α-olefins having at least (n/2 + 2) carbon atoms contained in the growth composition are bound to aluminum compounds with cleavage of α-olefins having fewer than (n/2 + 2) carbon atoms, preferably fewer than or equal to 6 carbon atoms, preferably by means of temperature increase and removal of the olefins with fewer than (n/2 + 2) carbon atoms from the reaction mixture.

13. The method according to claim 12, **characterized in that** the aluminum compounds that react with cleavage of α-olefins having fewer than (n/2 + 2) are di(*iso* C3- to C6- alkyl)aluminum hydride or tri(*iso* C3- to C6- alkyl)aluminum, preferably di(*iso*-butyl)aluminum hydride or tri(*iso*-butyl)aluminum, and that these are optionally added.

14. The method according to any of the previous claims, **characterized in that** the aluminum oxy compounds are distilled or stripped prior to hydrolysis in order to remove side products, such as paraffins, olefins, and/or esters, from the product mixture, while the aluminum oxy compounds remain in the bottom fraction.

15. The method according to any of the previous claims, **characterized in that** the growth reaction is carried out at 100 to 130 °C and a pressure of 20 to 120 bar.

16. The method according to any of the previous claims, **characterized in that** the separation in accordance with step (e) occurs by distillation, and that by means of distillation, an alcohol fraction is obtained that contains more than 90 mol%, more preferably more than 95 mol%, of linear alcohols.

17. The method according to any of claims 1 to 15, **characterized in that** as starting compounds, the following aluminum compounds are used:
(I) starting compounds that each have at least one hydrocarbon group having greater than or equal to 12 carbon atoms, preferably at least 2, and more preferably 3 groups, for the preparation of an alcohol fraction having on average 20 carbon atoms;
(II) starting compounds that each have at least one hydrocarbon group having greater than or equal to 14 carbon atoms, preferably at least 2, and more preferably 3 groups, for the preparation of an alcohol fraction having on average 20 to 24 carbon atoms;
(III) starting compounds that each have at least one hydrocarbon group having greater than or equal to 16 carbon atoms, preferably at least 2, and more preferably 3 groups, for the preparation of an alcohol fraction having on average 20 to 28 carbon atoms;
(IV) starting compounds that each have at least one hydrocarbon group having greater than or equal to 18 carbon atoms, preferably at least 2, and more preferably 3 groups, for the preparation of an alcohol fraction having on average 20 to 32 carbon atoms; or
(V) starting compounds that each have at least one hydrocarbon group having greater than or equal to 20 carbon atoms, preferably at least 2, and more preferably 3 groups, for the preparation of an alcohol fraction having on average 20 to 36 carbon atoms;
wherin the separation in accordance with step (e) in made by distillation; and
an alcohol fraction is obtained by means of distillation, that contains more than 90 mol%, more preferably more than 95 mol%, of linear alcohols.

18. The method according to any of claims 1 to 15, **characterized in that** as starting compounds, the following aluminum compounds are used:
(II) starting compounds that each have at least one hydrocarbon group having greater than or equal to 14 carbon atoms, preferably at least 2, and more preferably 3 groups, for the preparation of an alcohol fraction having on average 22 to 24 carbon atoms;
(III) starting compounds that each have at least one hydrocarbon group having greater than or equal to 16 carbon atoms, preferably at least 2, and more preferably 3 groups, for the preparation of an alcohol fraction having on average 26 to 28 carbon atoms;
(IV) starting compounds that each have at least one hydrocarbon group having greater than or equal to 18 carbon atoms, preferably at least 2, and more preferably 3 groups, for the preparation of an alcohol fraction having on average 30 to 32 carbon atoms; or
(V) starting compounds that each have at least one hydrocarbon group having greater than or equal to 20 carbon atoms, preferably at least 2, and more preferably 3 groups, for the preparation of an alcohol fraction having on average 34 to 36 carbon atoms;
wherein the separation in accordance with step (e) is made by distillation; and
an alcohol fraction is obtained by means of distillation, that contains more than 90 mol%, more preferably more than 95 mol%, of linear alcohols.

## Revendications

1. Procédé de préparation d'alcools primaires à longues chaînes ou de leurs mélanges avec, en moyenne, n atomes de carbone, n étant supérieur ou égal à 20, dans une réaction de croissance, ledit procédé comprenant les étapes suivantes consistant à :
(a) mettre à disposition des composés d'aluminium à titre de composés de départ de la réaction de croissance, lesquels présentent au moins un radical hydrocarboné comportant au moins (n/2+2) atomes de carbone, le radical hydrocarboné étant lié à l'atome d'aluminium à chaque fois par le biais d'un atome de carbone primaire et la composition des composés de départs de la réaction de croissance comprenant une teneur inférieure à 10 % en poids de 1-oléfines ayant plus de 2 atomes de carbone et moins de (n/2+2) atomes de carbone au total,
(b) mettre en contact par la suite les composés de départ dans une composition de croissance contenant le cas échéant d'autres composés d'aluminium, avec de l'éthylène pour la conversion avec de l'éthylène en formant au moins des composés d'aluminium à titre de composés de croissance, comportant au moins un radical hydrocarboné ayant, en moyenne, au moins z + (n/2+2) atomes de carbone à chaque fois par rapport au composé de départ, z étant supérieur ou égal à 2, les radicaux hydrocarbonés des composés de départ et de croissance qui comprennent au moins (n/2+2) atomes de carbone au total étant linéaires à au moins 90 % en moles,
(c) oxyder les produits réactionnels comprenant de l'éthylène avec de l'oxygène pour obtenir des composés aluminoxy comportant au moins un radical hydrocarboné lié à l'atome d'aluminium par le biais de l'oxygène,
(d) hydrolyser les composés aluminoxy, et
(e) séparer une composition contenant, en moyenne, des alcools comportant un nombre d'atomes de carbone supérieur ou égal à 20.

2. Procédé selon une des revendications précédentes, **caractérisé en ce que** la composition des composés de départ comporte, avant la réaction de croissance, une teneur inférieure à 3 % en poids de 1-oléfines avec plus de 2 et moins de (n/2+2) atomes de carbone au total.

3. Procédé selon une des revendications précédentes, **caractérisé en ce que** les radicaux hydrocarbonés des composés de départ et de croissance comportant au moins (n/2+2) atomes de carbone sont des radicaux alkyles.

4. Procédé selon une des revendications précédentes, **caractérisé en ce que** la composition comprend, après hydrolyse des composés aluminoxy (étape (d)), par rapport à tous les alcools présents dans la composition, au moins 4 % en poids d'alcools comportant plus de 20 atomes de carbone.

5. Procédé selon une des revendications 1 à 3, **caractérisé en ce que** la composition comprend, après hydrolyse des composés aluminoxy (étape (d)), par rapport à tous les alcools présents dans la composition, au moins 6 % en poids d'alcools comportant plus de 20 atomes de carbone.

6. Procédé selon une des revendications précédentes, **caractérisé en ce que** les alcools comportant plus de 20 atomes de carbone sont séparés par séparation de la phase aqueuse vis-à-vis de la phase organique et/ou par distillation.

7. Procédé selon une des revendications précédentes, **caractérisé en ce que** la teneur en 1-oléfines et/ou en radicaux hydrocarbonés liés à l'aluminium présents dans la composition de croissance, qui comportent respectivement moins de 24 atomes de carbone et sont ramifiés, est au total inférieure à 5 % en poids.

8. Procédé selon une des revendications précédentes, **caractérisé en ce que** les composés de départ sont préparés par échange de ligands contre des aluminoalkyles comportant des radicaux hydrocarbonés ayant 6 atomes de carbone ou moins en présence d'au moins une α-oléfine linéaire à longue chaîne ayant au moins (n/2+2) atomes de carbone.

9. Procédé selon une des revendications 1 à 7, **caractérisé en ce que** les composés de départ sont préparés par conversion de l'aluminium, de l'hydrogène et d'au moins une α-oléfine linéaire à longue chaîne ayant au moins (n/2+2) atomes de carbone.

10. Procédé selon une des revendications 1 à 7, **caractérisé en ce que** les composés de départ sont préparés par addition d'α-oléfines ayant au moins (n/2+2) atomes de carbone à un composé d'aluminium comportant au moins un radical hydrocarboné et au moins un atome d'eau lié par voie hydrique.

11. Procédé selon une des revendications précédentes, **caractérisé en ce que** les composés de départ sont cristallisés avant d'être ajoutés à la composition de croissance.

12. Procédé selon une des revendications précédentes, **caractérisé en ce que**, avant l'oxydation des produits réactionnels avec de l'oxygène, les α-oléfines comportant au moins (n/2+2) atomes de carbone, présentes dans la composition de croissance, sont liées aux composés d'aluminium en clivant hors du mélange réactionnel les α-oléfines ayant moins de (n/2+2) atomes de carbone, de préférence un nombre inférieur ou égal à 6 atomes de carbone, de préférence par hausse de la température et élimination des oléfines comportant moins de (n/2+2) atomes de carbone.

13. Procédé selon la revendication 12, **caractérisé en ce que** les composés d'aluminium qui réagissent en clivant les α-oléfines comportant moins de (n/2+2) atomes de carbone sont l'hydrure de di(isoalkyle en C₃ à C₆)aluminium ou tri(isoalkyle en C₃ à C₆)aluminium, de préférence l'hydrure de di(isobutyl)aluminium ou le tri(isobutyl)aluminium, et sont le cas échéant ajoutés.

14. Procédé selon une des revendications précédentes, **caractérisé en ce que** les composés d'aluminoxy sont distillés ou strippés avant l'hydrolyse pour éliminer du mélange de produits les sous-produits tels que les paraffines, les oléfines et/ou les esters alors que les composés aluminoxy restent dans le bas de colonne.

15. Procédé selon une des revendications précédentes, **caractérisé en ce que** la réaction de croissance est réalisée à une température de 100 à 130°C et à une pression de 20 à 120 bar.

16. Procédé selon une des revendications précédentes, **caractérisé en ce que** la séparation selon l'étape (e) se fait par distillation et une fraction alcoolique, qui comprend des alcools linéaires à plus de 90 % en moles, en particulier à plus de 95 % en moles, est obtenue par distillation.

17. Procédé selon une des revendications 1 à 15, **caractérisé en ce que** l'on met en oeuvre à titre de composés de départ les composés d'aluminium suivants
(I) les composés de départ comportant respectivement au moins un radical hydrocarboné ayant 12 atomes de carbone ou plus, de préférence au moins 2 et en particulier 3 radicaux, pour la préparation d'une fraction alcoolique ayant en moyenne 20 atomes de carbone ;
(II) les composés de départ comportant respectivement au moins un radical hydrocarboné ayant 14 atomes de carbone ou plus, de préférence au moins 2 et en particulier 3 radicaux, pour la préparation d'une fraction alcoolique ayant en moyenne 20 à 24 atomes de carbone ;
(III) les composés de départ comportant respectivement au moins un radical hydrocarboné ayant 16 atomes de carbone ou plus, de préférence au moins 2 et en particulier 3 radicaux, pour la préparation d'une fraction alcoolique ayant en moyenne 20 à 28 atomes de carbone ;
(IV) les composés de départ comportant respectivement au moins un radical hydrocarboné ayant 18 atomes de carbone ou plus, de préférence au moins 2 et en particulier 3 radicaux, pour la préparation d'une fraction alcoolique ayant en moyenne 20 à 32 atomes de carbone ; ou
(V) les composés de départ comportant respectivement au moins un radical hydrocarboné ayant 20 atomes de carbone ou plus, de préférence au moins 2 et en particulier 3 radicaux, pour la préparation d'une fraction alcoolique ayant en moyenne 20 à 36 atomes de carbone ;
dans lequel la séparation selon l'étape (e) s'effectue par distillation et
une fraction alcoolique qui comprend des alcools linéaires à plus de 90 % en moles, en particulier à plus de 95 % en moles, est obtenue par distillation.

18. Procédé selon une des revendications 1 à 15, **caractérisé en ce que** l'on met en oeuvre à titre de composés de départ les composés d'aluminium suivants
(II) les composés de départ comportant respectivement au moins un radical hydrocarboné ayant 14 atomes de carbone ou plus, de préférence au moins 2 et en particulier 3 radicaux, pour la préparation d'une fraction alcoolique ayant en moyenne 22 à 24 atomes de carbone ;
(III) les composés de départ comportant respectivement au moins un radical hydrocarboné ayant 16 atomes de carbone ou plus, de préférence au moins 2 et en particulier 3 radicaux, pour la préparation d'une fraction alcoolique ayant en moyenne 26 à 28 atomes de carbone ;
(IV) les composés de départ comportant respectivement au moins un radical hydrocarboné ayant 18 atomes de carbone ou plus, de préférence au moins 2 et en particulier 3 radicaux, pour la préparation d'une fraction alcoolique ayant en moyenne 30 à 32 atomes de carbone ; ou
(V) les composés de départ comportant respectivement au moins un radical hydrocarboné ayant 20 atomes de carbone ou plus, de préférence au moins 2 et en particulier 3 radicaux, pour la préparation d'une fraction alcoolique ayant en moyenne 34 à 36 atomes de carbone ;
dans lequel la séparation selon l'étape (e) s'effectue par distillation et
une fraction alcoolique qui comprend des alcools linéaires à plus de 90 % en moles, en particulier à plus de 95 % en moles, est obtenue par distillation.
